# EUROPEAN PATENT APPLICATION

(11) **EP 4 563 602 A1**
(43) Date of publication of application: **04.06.2025**
(21) Application number: 23846570.2
(22) Date of filing: 26.07.2023
(51) Int. Cl.: C08F 20/14, C08F 8/50, C08J 11/10

(54) **METHYL METHACRYLATE COMPOSITION**

(30) Priority: 29.07.2022 JP 2022121083
(71) Applicant: Sumitomo Chemical Company, Limited, Tokyo 103-6020 (JP)
(72) Inventor: YASUTOMI, Yoichi, Niihama-shi, Ehime 792-8521 (JP); KADOYA, Hidenori, Niihama-shi, Ehime 792-8521 (JP)
(74) Representative: Global IP Europe Patentanwaltskanzlei
(86) International application number: PCT/JP2023/027396
(87) International publication number: WO 2024/024840

(57) **Abstract**

The present invention addresses the problem of improving the heat resistance of a molded body.

The present invention pertains to a methyl methacrylate composition containing regenerative methyl methacrylate and non-regenerative methyl methacrylate, wherein: the content of the regenerative methyl methacrylate is 0.5-99.5 mass%; and the content of the non-regenerative methyl methacrylate is 0.5-99.5 mass%.

## Description

### TECHNICAL FIELD

The present invention relates to a methyl methacrylate composition containing methyl methacrylate as a main component.

### BACKGROUND ART

Polymethyl methacrylate (PMMA), which is a polymer obtained by polymerizing methyl methacrylate (MMA), is excellent in transparency and also in weather resistance. Therefore, polymethyl methacrylate is widely used as a material for members constituting automobile parts, signages, display devices, and the like.

As resource prices have increased in recent years and awareness of environmental problems has increased, polymethyl methacrylate products (molded bodies) used for various applications as described above have been recovered and recycled.

Examples of a method of recycling polymethyl methacrylate include material recycling in which a recovered molded body is subjected to a molding step again to produce a new molded body, chemical recycling in which methyl methacrylate is recovered by subjecting a recovered molded body to heat treatment to thermally decompose (depolymerize) polymethyl methacrylate, and a new molded body is produced using the recovered methyl methacrylate (may be referred to as recovered MMA), and thermal recycling in which the recovered molded body is combusted as fuel, and combustion energy is directly used as a heat source, and further, power is generated using combustion energy to be utilized.

It is preferable that polymethyl methacrylate is recycled by chemical recycling because methyl methacrylate can be recovered with a high yield and impurities can be reduced by heating at a relatively low temperature of about 300°C.

In chemical recycling using an acrylic-based resin waste material containing polymethyl methacrylate as a main component, there is known a technique in which, when depolymerization is performed by heat treatment using a heating pot having a stirring device, a monomer such as methyl methacrylate generated by the depolymerization is recovered as a high-purity monomer without further decomposition and change by focusing on a stirring blade tip speed and controlling the stirring blade tip speed (see Patent Document 1).

### PRIOR ART DOCUMENTS

### PATENT DOCUMENTS

Patent Document 1: JP-A-2003-321571

### SUMMARY OF THE INVENTION

### PROBLEMS TO BE SOLVED BY THE INVENTION

However, depending on methyl methacrylate obtained by the recovery method according to Patent Document 1, heat resistance of a molded body obtained by molding the methyl methacrylate composition containing methyl methacrylate may not be sufficient.

### MEANS FOR SOLVING THE PROBLEMS

As a result of intensive studies to solve the above problems, the present inventors have found that the above problems can be solved by controlling a composition of regenerated methyl methacrylate and non-regenerated methyl methacrylate in a methyl methacrylate composition, thereby completing the present invention.

That is, the present invention provides the following [1] to [12].
[1] A methyl methacrylate composition containing regenerated methyl methacrylate and non-regenerated methyl methacrylate,
   in which a content of the regenerated methyl methacrylate is 0.5 to 99.5% by mass, and a content of the non-regenerated methyl methacrylate is 0.5 to 99.5% by mass.
[2] The methyl methacrylate composition according to [1], in which the regenerated methyl methacrylate is regenerated methyl methacrylate derived from chemical recycling.
[3] The methyl methacrylate composition according to [1] or [2], in which the content of the regenerated methyl methacrylate is 5 to 40% by mass, and the content of the non-regenerated methyl methacrylate is 60 to 95% by mass.
[4] The methyl methacrylate composition according to any one of [1] to [3], further containing an alkyl acrylate.
[5] A method of producing a methyl methacrylate-based polymer, the method including polymerizing the methyl methacrylate composition according to any one of [1] to [3].
[6] A methyl methacrylate-based polymer obtained by polymerizing the methyl methacrylate composition according to any one of [1] to [3].
[7] A methyl methacrylate-based polymer containing a structural unit derived from regenerated methyl methacrylate and a structural unit derived from non-regenerated methyl methacrylate, in which a content of the structural unit derived from regenerated methyl methacrylate is 0.5 to 99.5% by mass, and a content of the structural unit derived from non-regenerated methyl methacrylate is 0.5 to 99.5% by mass.
[8] An agent for improving heat resistance for a methyl methacrylate polymer containing a regenerated methyl methacrylate product derived from chemical recycling from polymethyl methacrylate as an active ingredient, and containing non-regenerated methyl methacrylate.
[9] The agent according to [8], in which a polymer of the regenerated methyl methacrylate product has a 5% weight reduction temperature of 300°C or higher and/or a glass transition temperature of 90°C or higher.
[10] The agent according to [8] or [9], in which a polymer of the non-regenerated methyl methacrylate product has a 5% weight reduction temperature of 200°C or higher and/or a glass transition temperature of 105°C or higher.
[11] The agent according to any one of [8] to [10], in which a polymer of the regenerated methyl methacrylate product has a 5% weight reduction temperature higher than that of the non-regenerated methyl methacrylate product and/or a glass transition temperature lower than that of the non-regenerated methyl methacrylate product.
[12] A use for producing an agent for improving heat resistance for a methyl methacrylate polymer, the agent containing a regenerated methyl methacrylate product derived from chemical recycling from polymethyl methacrylate, and containing non-regenerated methyl methacrylate.

### EFFECT OF THE INVENTION

According to the present invention, it is possible to improve heat resistance of a methyl methacrylate-based polymer obtained by polymerizing a methyl methacrylate composition containing regenerated methyl methacrylate that may contain recovered MMA and non-regenerated methyl methacrylate, and a molded body thereof.

### BRIEF DESCRIPTION OF THE DRAWINGS

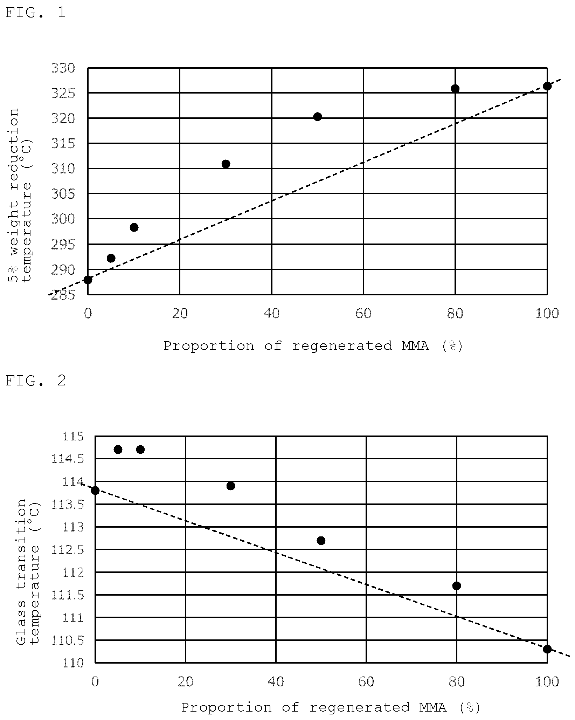

Fig. 1 is a graph showing a relationship between a proportion and a 5% weight reduction temperature of regenerated MMA in a molded body of a methyl methacrylate composition using recovered MMA of Preparation Example 1. The dotted line in Fig. 1 is a straight line connecting plots of Reference Example 1 and Comparative Example 1.
Fig. 2 is a graph showing a relationship between a proportion and a glass transition temperature of regenerated MMA in a molded body of a methyl methacrylate composition using recovered MMA of Preparation Example 1. The dotted line in Fig. 2 is a straight line connecting plots of Reference Example 1 and Comparative Example 1.

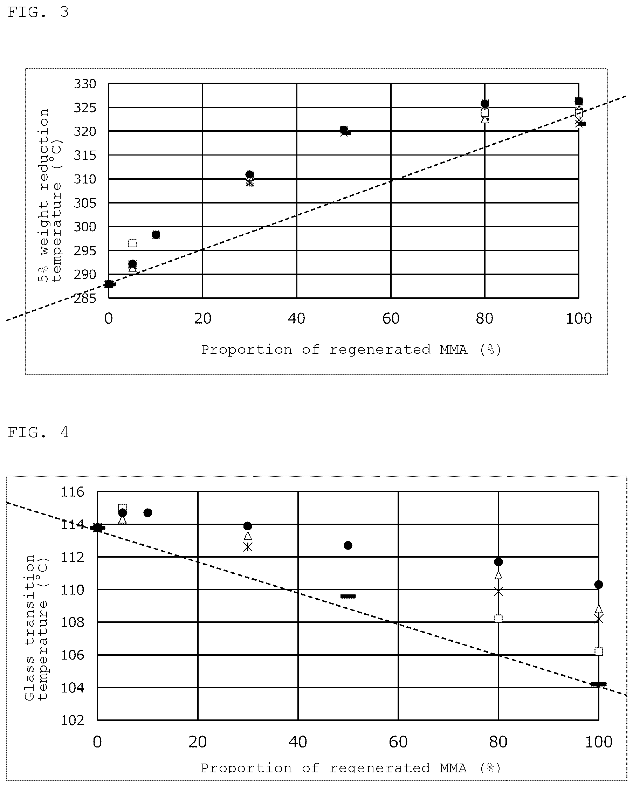

Fig. 3 is a graph showing a relationship between a proportion and a 5% weight reduction temperature of each regenerated MMA in a molded body of a methyl methacrylate composition using each of regenerated MMAs (A) to (D). The dotted line in Fig. 3 is a straight line connecting plots of Reference Example 1 and Comparative Example 2 of the regenerated MMA (A).
Fig. 4 is a graph showing a relationship between a proportion and a glass transition temperature of each regenerated MMA in a molded body of a methyl methacrylate composition using each of regenerated MMAs (A) to (D). The dotted line in Fig. 3 is a straight line connecting plots of Reference Example 1 and Comparative Example 5 of the regenerated MMA (D).

### MODE FOR CARRYING OUT THE INVENTION

Hereinafter, embodiments of the present invention will be described in detail. The present invention is not limited by the following description. In addition, each element according to the embodiments of the present invention can be appropriately changed without departing from the gist of the present invention.

### 1. Methyl Methacrylate Composition

A methyl methacrylate composition of the present embodiment contains regenerated methyl methacrylate and non-regenerated methyl methacrylate, in which of a content of the regenerated methyl methacrylate is 0.5 to 99.5% by mass and a content of the non-regenerated methyl methacrylate is 0.5 to 99.5% by mass.

According to the methyl methacrylate composition of the present embodiment, heat resistance, more specifically, a 5% weight reduction temperature, and further a glass transition temperature of a methyl methacrylate-based polymer obtained by polymerizing the methyl methacrylate composition and a molded body thereof can be improved, and a balance between the 5% weight reduction temperature and the glass transition temperature can be improved by achieving both the 5% weight reduction temperature and the glass transition temperature. In addition, the methyl methacrylate composition can exhibit excellent storability such as being able to maintain high quality even after long-term storage (for example, suppression of occurrence of yellowing). Therefore, even when the composition is stored for a long period of time after being prepared, high quality can be maintained, and the composition can be used for production of a molded body or the like in a timely manner.

The methyl methacrylate composition of the present embodiment is usually in a liquid form, but is not limited thereto. Hereinafter, components that may be contained in the methyl methacrylate composition of the present embodiment will be described in detail.

### (1) Methyl Methacrylate

"Methyl methacrylate" in the present embodiment refers to methyl methacrylate that is substantially free of impurities such as by-products such as methyl isobutyrate. However, the present invention is not limited to the definition on the proviso that the object of the present invention is not impaired. In other words, "methyl methacrylate" may contain impurities that cannot be removed by an ordinary purification method or may contain impurities in a content that cannot be detected by an ordinary detection method.

In the present embodiment, the method of producing methyl methacrylate is not particularly limited. Examples of a method of producing methyl methacrylate as a raw material of the methyl methacrylate composition of the present embodiment include "acetone cyanohydrin (ACH) method" in which acetone and hydrogen cyanide (hydrocyanic acid) are used as raw materials, "C4 direct oxidation method" in which isobutylene or tert-butyl alcohol is used as a raw material, and "alpha method" in which ethylene is used as a raw material.

As a raw material of the methyl methacrylate composition of the present embodiment, a product (may be referred to as "methyl methacrylate product") that may contain components other than methyl methacrylate, such as methyl isobutyrate, methyl isobutyrate, and/or methyl acrylate, can be used in a content that is mixed inevitably in the methyl methacrylate composition depending on the above production method.

Methyl methacrylate (or methyl methacrylate product) that is to be used as a raw material of the methyl methacrylate composition of the present embodiment and is produced by the above production method may be purified by any conventionally-known suitable purification method.

A content of methyl isobutyrate that may be contained in the methyl methacrylate product as a raw material of the methyl methacrylate composition of the present embodiment is preferably more than 0 ppm by mass and 300 ppm by mass or less, and/or a content of methyl acrylate that may be contained in the methyl methacrylate product is preferably 0 ppm by mass to 20,000 ppm by mass, when the total amount of the methyl methacrylate product is 100 parts by mass.

In the methyl methacrylate composition of the present embodiment, a content of the methyl methacrylate is usually 85% by mass or more, preferably 90% by mass or more, more preferably 95% by mass or more, still more preferably 98% by mass or more, and particularly preferably 99% by mass or more, when the total amount of the methyl methacrylate composition is 100% by mass, from the viewpoint of improving the heat resistance.

In the present embodiment, the methyl methacrylate composition contains regenerated methyl methacrylate and non-regenerated methyl methacrylate as methyl methacrylate. Hereinafter, regenerated methyl methacrylate and non-regenerated methyl methacrylate will be described.

### (1-1) Regenerated Methyl Methacrylate

In the present embodiment, "regenerated methyl methacrylate" refers to methyl methacrylate derived from chemical recycling regenerated by depolymerizing (chemical recycling) a molded body obtained by molding a methyl methacrylate composition containing polymethyl methacrylate, that is, methyl methacrylate, and further methyl methacrylate as a main component into various shapes by any conventionally-known suitable injection molding step or the like. Specifically, "regenerated methyl methacrylate" is, for example, polymethyl methacrylate obtained by polymerizing methyl methacrylate, and more specifically, methyl methacrylate (recovered MMA) recovered by thermally decomposing (depolymerizing) a molded body containing polymethyl methacrylate as a main component that is recovered after being distributed on the market and applied to various applications or before being distributed on the market (for example, in excess production) by heat treatment under any conventionally-known suitable conditions.

In the present embodiment, the embodiment of chemical recycling for obtaining regenerated methyl methacrylate is not particularly limited. In the present embodiment, the chemical recycling can be performed under any conventionally-known suitable conditions (thermal decomposition conditions) using any conventionally-known suitable device. For example, a thermal decomposition temperature can be, for example, about 300 to 600°C, preferably about 350 to 500°C, and more preferably about 400 to 500°C. When an extruder is used for thermal decomposition, regenerated methyl methacrylate that can be used in the composition of the present embodiment can be obtained within the above range.

By performing depolymerization of polymethyl methacrylate (for example, chemical recycling), a regenerated methyl methacrylate product containing regenerated methyl methacrylate is usually obtained. Examples of components other than regenerated methyl methacrylate contained in the regenerated methyl methacrylate product include methyl isobutyrate, methyl propionate, methyl acrylate, and other components derived from depolymerization of polymethyl methacrylate, that is, chemical recycling.

According to the chemical recycling, regenerated methyl methacrylate can be recovered with a high yield. In addition, any conventionally-known suitable purification step is performed after the depolymerization, such that it is possible to effectively reduce a content (concentration) of low-boiling-point compounds (impurities) that are by-products, for example, methyl propionate, methyl isobutyrate, and methyl acrylate, that may be inevitably contained in a regenerated methyl methacrylate product containing produced methyl methacrylate as a main component. The purification step may be performed at a temperature at which impurities can be reduced, and may be performed, for example, in a range of 30 to 130°C and preferably 30 to 120°C. Note that the purification step may be performed under reduced pressure conditions as long as regenerated methyl methacrylate can be recovered. When the purification step is performed under a reduced pressure environment, the purification step may be performed at a temperature lower than 100°C, preferably in a range of 30 to 80°C, and more preferably 50 to 80°C.

The regenerated methyl methacrylate product usually contains 80% by mass or more, preferably 85% by mass or more, more preferably 90% by mass or more, still more preferably 92% by mass or more, and still more preferably 95% by mass or more of regenerated methyl methacrylate. An upper limit of the content of the regenerated methyl methacrylate is not particularly limited, and may be 100% by mass, 99% by mass or less, or 98% by mass or less. That is, the regenerated methyl methacrylate product contains regenerated methyl methacrylate usually in a range of 80 to 100% by mass and preferably in a range of 85 to 99% by mass.

Although regenerated methyl methacrylate is basically methyl methacrylate having the same chemical structure as that of non-regenerated methyl methacrylate, it has been found that the physical properties of the composition or the polymer are changed when regenerated methyl methacrylate is added to non-regenerated methyl methacrylate. The reason for this is not clear, but it is presumed that the component is a separate component which undergoes thermal decomposition (depolymerization) in a process of producing regenerated methyl methacrylate, and thus, changes reactivity from non-regenerated methyl methacrylate for some reason, and exhibits behavior different from that of non-regenerated methyl methacrylate. As such a reason, disappearance of at least a part of the reaction point, a change in purity, a change in interaction between monomers, and the like are considered.

### (1-2) Non-Regenerated Methyl Methacrylate

In the present embodiment, "non-regenerated methyl methacrylate" refers to methyl methacrylate that does not correspond to "regenerated methyl methacrylate" described above, and specifically refers to, for example, methyl methacrylate that has not been polymerized and applied to various applications after being produced by a predetermined production method (virgin methyl methacrylate).

As described above, the non-regenerated methyl methacrylate may inevitably contain other components (impurities) such as methyl isobutyrate, methyl propionate, and methyl acrylate. Such a composition containing non-regenerated methyl methacrylate and other components may be referred to as a non-regenerated methyl methacrylate product. The content of the non-regenerated methyl methacrylate in the non-regenerated methyl methacrylate product is usually 95% by mass or more, preferably 96% by mass or more, more preferably 97% by mass or more, still more preferably 98% by mass or more, and still more preferably 99% by mass or more. An upper limit thereof is not particularly limited, and may be 100% by mass. That is, the content of the non-regenerated methyl methacrylate in the non-regenerated methyl methacrylate product is usually in a range of 95 to 100% by mass and preferably in a range of 96 to 99% by mass.

In the present embodiment, a method of producing non-regenerated methyl methacrylate to be used as a raw material of the methyl methacrylate composition is not particularly limited. Examples of the method of producing non-regenerated methyl methacrylate to be used as a raw material of the methyl methacrylate composition of the present embodiment include "acetone cyanohydrin (ACH) method", "C4 direct oxidation method", and "alpha method" described above. In the present embodiment, the non-regenerated methyl methacrylate may be produced by any of these methods.

In the methyl methacrylate composition of the present embodiment, the non-regenerated methyl methacrylate includes methyl methacrylate (may be referred to as "bio-MMA") produced using a plant-derived raw material. Specifically, the bio-MMA may include methyl methacrylate produced by the production method such as the acetone cyanohydrin (ACH) method, the C4 direct oxidation method, or the alpha method described above, using butanol, isobutylene, ethanol, ethylene, methanol, propylene acetone, or the like produced by decomposition, fermentation, or the like by microorganisms using a plant-derived raw material such as starch or polysaccharides derived from plants such as corn, wheat, and potato.

In the present embodiment, for example, methyl methacrylate produced by the above production method can be included in "bio-MMA" as long as a raw material, a reagent, or the like produced by the action of decomposition, fermentation, or the like by microorganisms is used.

Note that bio-MMA may contain carbon 14 (¹⁴C), which is a radioactive isotope of carbon 12 (¹²C), whereas methyl methacrylate derived from a mineral resource such as petroleum does not contain carbon 14 (¹⁴C). Therefore, whether or not the non-regenerated methyl methacrylate, further the regenerated methyl methacrylate, the methyl methacrylate composition, and the molded body obtained by molding the methyl methacrylate composition contain bio-MMA or are derived from bio-MMA can be confirmed by detecting whether or not these components contain carbon 14 (¹⁴C) by any conventionally-known suitable method.

The non-regenerated methyl methacrylate produced by the above production method can effectively reduce the content (concentration) of impurities that may be inevitably contained in the non-regenerated methyl methacrylate product containing the produced methyl methacrylate as a main component by performing any conventionally-known suitable purification step.

In the methyl methacrylate composition of the present embodiment, as for the contents of the regenerated methyl methacrylate and the non-regenerated methyl methacrylate, it is preferable that the content of the regenerated methyl methacrylate is 0.5 to 99.5% by mass, and the content of the non-regenerated methyl methacrylate is 0.5 to 99.5% by mass, when the total amount of the methyl methacrylate composition is 100% by mass, from the viewpoint of achieving a balance between the 5% weight reduction temperature and the glass transition temperature and suppressing the occurrence of yellowing. Regarding these contents, it is more preferable that the content of the regenerated methyl methacrylate is 3 to 90% by mass and the content of the non-regenerated methyl methacrylate is 10 to 97% by mass, it is still more preferable that the content of the regenerated methyl methacrylate is 5 to 80% by mass and the content of the non-regenerated methyl methacrylate is 20 to 95% by mass, it is still more preferable that the content of the regenerated methyl methacrylate is 10 to 70% by mass and the content of the non-regenerated methyl methacrylate is 30 to 90% by mass, and it is still more preferable that the content of the regenerated methyl methacrylate is 20 to 60% by mass and the content of the non-regenerated methyl methacrylate is 40 to 80% by mass. When the contents of the regenerated methyl methacrylate and the non-regenerated methyl methacrylate are within the above ranges, the heat resistance of the polymer of the methyl methacrylate composition and the molded body thereof can be improved, and the occurrence of yellowing of the methyl methacrylate composition can be suppressed, which can improve the storage stability. In addition, in another embodiment, it is more preferable that the content of the regenerated methyl methacrylate is 3 to 40% by mass and the content of the non-regenerated methyl methacrylate is 60 to 97% by mass, and it is still more preferable that the content of the regenerated methyl methacrylate is 3 to 20% by mass and the content of the non-regenerated methyl methacrylate is 80 to 97% by mass.

### (2) Alkyl Acrylate

The methyl methacrylate composition of the present embodiment may further contain an alkyl acrylate. The alkyl acrylate that may be contained in the methyl methacrylate composition of the present embodiment can be methyl acrylate produced by the method of producing methyl methacrylate described above and inevitably mixed, or methyl acrylate inevitably produced by depolymerization related to the chemical recycling described above. In addition, the alkyl acrylate that may be contained in the methyl methacrylate composition of the present embodiment can be an alkyl acrylate that is intentionally mixed with the above methyl methacrylate product (the regenerated methyl methacrylate product or the non-regenerated methyl methacrylate product). Examples of the alkyl acrylate include methyl acrylate, ethyl acrylate, propyl acrylate, and butyl acrylate.

A content of the alkyl acrylate that may be contained in the methyl methacrylate composition of the present embodiment is preferably 0% by mass to 10% by mass, and more preferably 0% by mass to 5% by mass.

A concentration of methyl acrylate that may be contained in the methyl methacrylate composition of the present embodiment is preferably 5 ppm by mass to 50,000 ppm by mass and more preferably 5 ppm by mass to 30,000 ppm by mass.

### (3) Other Low-Content Components

The methyl methacrylate composition of the present embodiment may contain, in addition to the "impurities" described above, other low-content components particularly resulting from recovering of regenerated methyl methacrylate. Examples of the other low-content components include methanol, methyl 2,4-dimethyl-4-pentenoate, 1-butanol, and butyl acrylate, and may further contain methyl 2-methyl-3-butenoate, methyl tiglate, methyl 3-methyl-3-butenoate, methyl 3-methyl-2-butenoate, dimethyl itaconate, dimethyl 2-methyl-5-methylenehexanedioate, toluene, and styrene.

When the methyl methacrylate composition of the present embodiment further contains one or two or more selected from the group consisting of methanol, methyl 2,4-dimethyl-4-pentenoate, 1-butanol, and butyl acrylate among the above other low-content components, a concentration of each of the methanol, methyl 2,4-dimethyl-4-pentenoate, 1-butanol, and butyl acrylate is preferably 10 ppm by mass to 5,000 ppm by mass, and more preferably 10 ppm by mass to 4,000 ppm by mass, when the total amount of the methyl methacrylate composition is 100 parts by mass.

When the methyl methacrylate composition of the present embodiment further contains one or two or more selected from the group consisting of methyl 2-methyl-3-butenoate, methyl tiglate, methyl 3-methyl-3-butenoate, methyl 3-methyl-2-butenoate, dimethyl itaconate, dimethyl 2-methyl-5-methylenehexanedioate, toluene, and styrene among the above other low-content components, a concentration of each of the methyl 2-methyl-3-butenoate, methyl tiglate, methyl 3-methyl-3-butenoate, methyl 3-methyl-2-butenoate, dimethyl itaconate, dimethyl 2-methyl-5-methylenehexanedioate, toluene, and styrene is preferably 10 ppm by mass to 1,000 ppm by mass, and more preferably 10 ppm by mass to 800 ppm by mass, when the total amount of the methyl methacrylate composition is 100 parts by mass.

According to the methyl methacrylate composition of the present embodiment, even when the above other low-content components are further contained at the above concentrations, a 5% weight reduction temperature, and further a glass transition temperature of a methyl methacrylate-based polymer obtained by polymerizing the methyl methacrylate composition, and further, a molded body thereof can be improved, and a balance between the 5% weight reduction temperature and the glass transition temperature can be improved by achieving both the 5% weight reduction temperature and the glass transition temperature. Specifically, for example, the 5% weight reduction temperature and the glass transition temperature of the methyl methacrylate composition of the present embodiment can show values much higher than predicted from the 5% weight reduction temperature and the glass transition temperature predicted from non-regenerated methyl methacrylate or regenerated methyl methacrylate alone. For example, when the relationship between the measured values of the 5% weight reduction temperature of the above single molded product and the composition of the present embodiment and the content of the regenerated methyl methacrylate is plotted, and the measured values of the single molded product are connected by a straight line, the methyl methacrylate composition of the present embodiment can show a measured value exceeding the straight line. In addition, the same applies to the glass transition temperature.

In addition, according to the methyl methacrylate composition of the present embodiment, excellent storage properties can be exhibited, and deterioration such as yellowing during storage can be suppressed. Since the non-regenerated methyl methacrylate and the regenerated methyl methacrylate are deteriorated during storage, the effect obtained by mixing both at a predetermined ratio can be said to be an unexpected and surprising effect. Although the reason is not clear, it is considered that some actions (for example, interactions between monomers and the like) different from the case of only the non-regenerated methyl methacrylate or the case of only the regenerated methyl methacrylate occur by adding regenerated methyl methacrylate to non-regenerated methyl methacrylate.

### (4) Other Components

On the proviso that the object of the present invention is not impaired, in addition to the above components (1) to (3), the methyl methacrylate composition of the present embodiment may contain other components such as a release agent, a polymerization modifier, a polymerization initiator, an ultraviolet absorber, and a colorant that may be added to produce a molded body having predetermined properties. Hereinafter, other components that may be contained in the methyl methacrylate composition of the present embodiment will be described.

### (i) Release Agent

In production of a methyl methacrylate-based polymer obtained by polymerizing a methyl methacrylate composition and a molded body thereof, the methyl methacrylate composition of the present embodiment may particularly contain a release agent for improving releasability of the molded body.

Examples of the release agent that may be contained in the methyl methacrylate composition of the present embodiment include a higher fatty acid ester, a higher aliphatic alcohol, a higher fatty acid, a higher fatty acid amide, a higher fatty acid metal salt, and a fatty acid derivative.

Specific examples of the release agent that may be contained in the methyl methacrylate composition of the present embodiment include sodium di-(2-ethylhexyl) sulfosuccinate, stearyl alcohol, methyl stearate, and stearic acid amide. In the methyl methacrylate composition of the present embodiment, these exemplified release agents may be used alone or in combination of two or more kinds thereof.

A content of the release agent in the methyl methacrylate composition of the present embodiment is, for example, 0.01 parts by mass to 1.0 parts by mass when the total amount of the methyl methacrylate composition is 100 parts by mass.

### (ii) Polymerization Modifier

The methyl methacrylate composition of the present embodiment may contain a polymerization modifier that can control a polymerization rate in a polymerization reaction of methyl methacrylate.

As the polymerization modifier that may be contained in the methyl methacrylate composition of the present embodiment, it is possible to use any conventionally-known suitable polymerization modifier. Examples of the polymerization modifier include compounds that can control a polymerization rate so as to reduce the polymerization rate.

Specific preferred examples of the polymerization modifier include mercaptan compounds such as n-butyl mercaptan and n-octyl mercaptan; terpenoid compounds such as limonene, myrcene, α-terpinene, β-terpinene, **γ-**terpinene, terpinolene, β-pinene, and α-pinene; and an α-methylstyrene dimer. In the present embodiment, as the polymerization modifier, terpinolene, which is a terpenoid compound, is preferably used.

In the methyl methacrylate composition of the present embodiment, these exemplified polymerization modifiers may be used alone or in combination of two or more kinds thereof.

A content of the polymerization modifier in the methyl methacrylate composition of the present embodiment is, for example, 0.001 parts by mass to 0.5 parts by mass when the total amount of the methyl methacrylate composition is 100 parts by mass.

### (iii) Polymerization Initiator

Examples of the polymerization initiator that may be contained in the methyl methacrylate composition of the present embodiment include a radical polymerization initiator, a diacyl peroxide initiator, a dialkyl peroxide initiator, a peroxyester initiator, a percarbonate initiator, and a peroxyketal initiator.

Specific examples of the radical polymerization initiator include azo compounds such as 1,1'-azobis(cyclohexane-1-carbonitrile), 2,2'-azobis(2,4,4-trimethylpentene), 2,2'-azobis(2-methylpropane), 2-cyano-2-propylazoformamide, 2,2'-azobis(2-hydroxy-methylpropionate), 2,2'-azobis(2-methyl-butyronitrile), 2,2'-azobisisobutyronitrile, 2,2'-azobis[2-(2-imidazolin-2-yl)propane], and dimethyl 2,2'-azobis(2-methyl propionate).

Specific examples of the diacyl peroxide initiator and the dialkyl peroxide initiator include dicumyl peroxide, tert-butyl cumyl peroxide, di-tert-butyl peroxide, benzoyl peroxide, and lauroyl peroxide.

Specific examples of the peroxyester initiator include tert-butyl peroxy-3,3,5-trimethylhexanoate, tert-butyl peroxylaurate, tert-butyl peroxyisobutyrate, tert-butyl peroxyacetate, di-tert-butyl peroxyhexahydroterephthalate, di-tert-butyl peroxyazelate, tert-butyl peroxy-2-ethylhexanoate, 1,1,3,3-tetramethylbutyl peroxy-2-ethylhexanoate, and tert-amylperoxy-2-ethylhexanoate.

Specific examples of the percarbonate initiator include tert-butylperoxyallyl carbonate and tert-butylperoxyisopropyl carbonate.

Specific examples of the peroxyketal initiator include 1,1-di-tert-butylperoxycyclohexane, 1,1-di-tert-butylperoxy-3,3,5-trimethylcyclohexane, and 1,1-di-tert-hexylperoxy-3,3,5-trimethylcyclohexane.

In the methyl methacrylate composition of the present embodiment, these exemplified polymerization initiators may be used alone or in combination of two or more kinds thereof.

A content of the polymerization initiator in the methyl methacrylate composition of the present embodiment is, for example, 0.01 parts by mass to 5 parts by mass when the total amount of the methyl methacrylate composition is 100 parts by mass.

### (iv) Ultraviolet Absorber

The methyl methacrylate composition of the present embodiment may contain an ultraviolet absorber in order to further improve weather resistance of the polymethyl methacrylate to be produced and a molded body thereof.

Preferred examples of the ultraviolet absorber that may be contained in the methyl methacrylate composition of the present embodiment include a benzophenone ultraviolet absorber, a cyanoacrylate ultraviolet absorber, a benzotriazole ultraviolet absorber, a malonic acid ester ultraviolet absorber, and an oxalanilide ultraviolet absorber.

Specific examples of the ultraviolet absorber include 2-(2'-hydroxy-5'-methylphenyl)benzotriazole, 2-hydroxy-4-n-octylbenzophenone, 2-(3-tert-butyl-2-hydroxy-5-methylphenyl)-5-chlorobenzotriazole, 2-(2-hydroxy-3,5-di-tert-pentylphenyl)benzotriazole, and 2,4-di-tert-butylphenyl-3,5-di-tert-butyl-4-hydroxybenzoate.

In the methyl methacrylate composition of the present embodiment, these exemplified ultraviolet absorbers may be used alone or in combination of two or more kinds thereof.

A content of the ultraviolet absorber in the methyl methacrylate composition of the present embodiment is, for example, 0.001 parts by mass to 1 part by mass when the total amount of the methyl methacrylate composition is 100 parts by mass.

### (v) Colorant

The methyl methacrylate composition of the present embodiment may contain a colorant in order to turn a methyl methacrylate-based polymer to be produced and a molded body thereof into any suitable color.

Preferred examples of the colorant that may be contained in the methyl methacrylate composition of the present embodiment include a perylene dye, a perinone dye, a pyrazolone dye, a methine dye, a coumarin dye, a quinophthalone dye, a quinoline dye, an anthraquinone dye (for example: Sumiplast Violet B), an anthraquinone dye, an anthrapyridone dye, a thioindigo dye, a coumarin dye, an isoindolinone pigment, a diketopyrrolopyrrole pigment, a condensed azo pigment, a benzimidazolone pigment, a dioxazine pigment, a copper phthalocyanine pigment, and a quinacridone pigment.

These exemplified colorants may be used alone or in combination of two or more kinds thereof.

A content of the colorant in the methyl methacrylate composition of the present embodiment is, for example, 1.0 × 10⁻⁸ parts by mass to 0.5 parts by mass when the total amount of the methyl methacrylate-based composition is 100 parts by mass.

### 2. Method of Producing Methyl Methacrylate Composition

The methyl methacrylate composition of the present embodiment can be produced by mixing the components described above under any suitable conditions by any conventionally-known suitable method.

Specifically, the methyl methacrylate composition of the present embodiment can be produced by preparing the components described above in a predetermined amount and mixing the components in a mixing tank controlled at a constant temperature with a heat medium jacket or the like having any conventionally-known suitable configuration.

The methyl methacrylate composition of the present embodiment may be obtained by purifying the methyl methacrylate composition (mixture) produced by the above production method by any conventionally-known suitable purification method.

In this manner, according to the method of producing a methyl methacrylate composition of the present embodiment, it is possible to easily produce a methyl methacrylate composition that enables production of a methyl methacrylate-based polymer having the excellent properties described above, and a molded body thereof at low cost.

### 3. Method of Producing Methyl Methacrylate-Based Polymer and Molded Body Thereof

In the present embodiment, a method of producing a methyl methacrylate-based polymer includes polymerizing a methyl methacrylate composition. A method of forming the methyl methacrylate composition of the present embodiment into a methyl methacrylate-based polymer is not particularly limited. Specifically, the methyl methacrylate composition can be polymerized into a methyl methacrylate-based polymer by any suitable method selected in consideration of components contained in the methyl methacrylate composition of the present embodiment and contents of the components.

In the present embodiment, examples of the method of polymerizing the methyl methacrylate composition include methods such as bulk polymerization, solution polymerization, suspension polymerization, and emulsion polymerization.

Specifically, a molded body of a methyl methacrylate-based polymer, that is, a molded body containing polymethyl methacrylate can be obtained by polymerizing the methyl methacrylate composition of the present embodiment by, for example, a bulk polymerization method. In addition, in the cell cast polymerization, any suitable heat treatment can be performed on the methyl methacrylate composition under predetermined heating conditions to advance polymerization reactions. Accordingly, a molded body of a methyl methacrylate-based polymer obtained by polymerizing and curing methyl methacrylate contained in the methyl methacrylate composition can be obtained.

In the method of polymerizing a methyl methacrylate composition (methyl methacrylate) of the present embodiment to obtain a methyl methacrylate-based polymer, and further in the method of producing a molded body molded by polymerizing a methyl methacrylate composition, a heating temperature and a heating time in the heating conditions can be appropriately set in consideration of, for example, the types and contents of the selected polymerization modifier, polymerization initiator, and/or other components, the content of methyl methacrylate, and the concentrations of the alkyl acrylate and other components that may be contained.

In the present embodiment, in the cell cast polymerization, a heating temperature is set to, for example, 50°C to 130°C and a heating time can be set to, for example, 1 hour to 20 hours to produce a methyl methacrylate-based polymer, that is, a molded body.

The heat treatment in the method of producing a polymer and a molded body of the present embodiment can involve a plurality of steps performed at different heating temperatures and/or heating times.

The polymer of the present embodiment and a molded body thereof can be produced by the heat treatment under heating conditions including, for example, the following Steps 1 to 7.

Step 1: Raise the temperature from room temperature to 68°C for 20 minutes.

Step 2: Hold the temperature at 68°C for 90 minutes.

Step 3: Lower the temperature from 68°C to 64°C for 20 minutes.

Step 4: Hold the temperature at 64°C for 90 minutes.

Step 5: Raise the temperature from 64°C to 123°C for 10 minutes.

Step 6: Hold the temperature at 123°C for 120 minutes.

Step 7: Lower the temperature from 123°C to room temperature for 90 minutes.

In the method of producing a methyl methacrylate-based polymer and a molded body of the present embodiment, Steps 1 to 7 are performed in this order, such that it possible to suppress heat generation in the polymerization reaction and to terminate the polymerization reaction stably.

In the heat treatment of the methyl methacrylate composition of the present embodiment, for example, a cell casting method (cell cast polymerization) is applied using a cell that defines a predetermined-shaped sealed space inside the cell, thereby forming a molded body with a predetermined shape. Hereinafter, a method of producing a molded body by the cell casting method will be described in detail.

In producing a molded body of a methyl methacrylate-based polymer by the cell casting method, first, a cell is prepared. Here, a plate-shaped molded body (may be referred to as "cast plate") will be described.

Such a cell can include at least two flat-plate members and a seal material (gasket) that is sandwiched between the two flat-plate members and can seal a gap between the two flat-plate members facing each other to form a sealed space.

The flat-plate member may have the form of a sheet or belt. The flat-plate member may include a material that is not dissolved by the methyl methacrylate composition of the present embodiment, does not inhibit polymerization reactions, and has sufficient heat resistance to the heating temperature during the heat treatment. Preferred examples of the material of the flat-plate member include glass and a metal.

As the seal material, any conventionally-known suitable seal material may be used. The seal material may include a material that is not dissolved by the methyl methacrylate composition of the present embodiment, does not inhibit polymerization reactions, and has sufficient heat resistance to the heating temperature during the heat treatment. Specific preferred examples of the seal material include a vinyl chloride resin gasket.

Next, the methyl methacrylate composition of the present embodiment is injected into a gap (void) defined by the cell prepared as described above by any conventionally-known suitable method.

Next, the cell is subjected to heat treatment under the above heating conditions. The heat treatment for the cell to which the methyl methacrylate composition of the present embodiment is injected is not particularly limited. Similarly to the conventionally-known cell casting method, the heat treatment method for the cell can be a method of directly performing heat treatment from the outside of the cell using, for example, a hot air circulation furnace or an infrared heater, and a method of further providing any conventionally-known suitable jacket outside the cell and introducing a heat medium such as hot air, hot water, and water vapor into the jacket.

### 4. Measurement of 5% Weight Reduction Temperature (Heating Weight Reduction Percentage) of Molded Body

In the present embodiment, the 5% weight reduction temperature of the molded body of the methyl methacrylate-based polymer can be measured using a conventionally known thermogravimetric/differential thermal analyzer (for example, "TG/DTA7200" manufactured by Hitachi High-Tech Science Corporation).

Specifically, a test piece that is a pulverized product obtained by pulverizing a molded body such as a cast plate so as to have a diameter or a length of each side of 0.5 mm or less is subjected to heat treatment of raising the temperature to a predetermined temperature at a predetermined gas flow rate and a heating rate under circulation of an inert gas using a thermogravimetric/differential thermal analyzer, and the weight of the heat-treated test piece is measured, such that the 5% weight reduction temperature can be calculated based on the measured weight and the initial weight.

In the present embodiment, the 5% weight reduction temperature of the molded body, that is, the methyl methacrylate-based polymer is preferably 260°C to 360°C, and more preferably 290°C to 330°C.

When the 5% weight reduction temperature of the methyl methacrylate-based polymer is lower than 260°C, a molded body obtained using the methyl methacrylate-based polymer may have poor appearance. On the other hand, when the 5% weight reduction temperature of the methyl methacrylate-based polymer is higher than 330°C, large energy may be required for thermal decomposition of a molded body obtained using the methyl methacrylate-based polymer.

In the regenerated methyl methacrylate product as a raw material of the composition, the 5% weight reduction temperature of a molded body obtained using the homopolymer (regenerated methyl methacrylate polymer) thereof is usually 300°C or higher, preferably 310°C or higher, and more preferably 320°C or higher. An upper limit thereof is usually 400°C or lower, preferably 380°C or lower, and more preferably 350°C or lower. In the non-regenerated methyl methacrylate product as a raw material, the 5% weight reduction temperature of a molded body obtained using the homopolymer (non-regenerated methyl methacrylate polymer) thereof is usually 200°C or higher and preferably 250°C or higher. An upper limit thereof is usually lower than 300°C and preferably 290°C or lower. The 5% weight reduction temperature of the molded body of the regenerated methyl methacrylate polymer is preferably higher than that of the molded body of the non-regenerated methyl methacrylate polymer, and the difference is usually 10°C or higher, preferably 20°C or higher, and more preferably 30°C or higher. An upper limit thereof is usually 50°C or lower and preferably 40°C or lower.

### 5. Measurement of Glass Transition Temperature

In the present embodiment, a glass transition temperature of the molded body of the methyl methacrylate-based polymer is a glass transition temperature (Tmg) (°C) measured in accordance with JIS-K7121.

The glass transition temperature can be measured using any conventionally-known suitable measurement apparatus (for example, "DSC7020", manufactured by Hitachi High-Tech Science Corporation, which is a differential scanning calorimeter).

Specifically, using a test piece that is a pulverized product obtained by pulverizing a molded body such as a cast plate so that a diameter or a length of each side is 0.5 mm or less, the measurement can be performed based on a DSC curve obtained by a predetermined temperature profile after the test piece is completely melted in an inert gas atmosphere.

In the present embodiment, the glass transition temperature of the molded body, that is, the methyl methacrylate-based polymer is usually 100°C to 130°C or 105°C to 125°C, preferably 110°C to 120°C, and more preferably 110°C to 115°C.

The glass transition temperature of the polymer (molded body) of regenerated methyl methacrylate is usually 90°C or higher, preferably 95°C or higher, more preferably 100°C or higher, and still more preferably 105°C or higher. An upper limit thereof is usually 130°C or lower, preferably 120°C or lower, and more preferably 110°C or lower. The glass transition temperature of the polymer (molded body) of non-regenerated methyl methacrylate is usually 105°C or higher and preferably 110°C or higher. An upper limit thereof is usually 150°C or lower and preferably 140°C or lower. The glass transition temperature of the regenerated methyl methacrylate polymer (molded body) is preferably higher than that of the non-regenerated methyl methacrylate polymer (molded body), and the difference is usually 2°C or higher, preferably 2.5°C or higher, and more preferably 3°C or higher. An upper limit of the heating temperature is usually 10°C or lower, preferably 9°C or lower, and more preferably 8°C or lower.

### 6. Measurement of Yellow Index

A yellow index (YI value) of the molded body of the methyl methacrylate-based polymer in the present embodiment can be calculated according to the method described in JIS K7373. In examples described below, a composition to be measured is placed in a quartz glass cell of 10 mm in length × 10 mm in width × 45 mm in height, and a yellow index (YI value) when an optical path length is 10 mm is calculated according to the method described in JIS K7373. For the measurement, a commercially available spectrophotometer can be used, and for example, the measurement may be performed under the following conditions. The smaller the calculated YI value is, the more the yellowing is suppressed. For example, the YI value measured under the measurement conditions of the examples is preferably 0.1 or less.

### (YI Measurement Condition)

Light source: C light source
Field of view: 2 degrees Calculation Formula: YI = (1.2769 × X - 1.0592 × Z)/Y × 100

### 7. Use of Polymer and Molded Body Thereof

A polymer of the methyl methacrylate composition of the present embodiment and a molded body thereof are excellent in light permeability, heat resistance, and weather resistance. Therefore, the present invention can be suitably applied to various applications such as lighting equipment, automobile parts, signboards, and building materials that may be exposed to external environments and also to heat sources and light sources.

### EXAMPLES

Examples according to embodiments of the present invention will be described. The present invention is not limited by the following Examples.

### <Preparation Example 1>

### · Preparation of regenerated methyl methacrylate product (recovered MMA)

A polymethyl methacrylate resin (SUMIPEX MH, manufactured by Sumitomo Chemical Co., Ltd.) was thermally decomposed (depolymerized) using a twin-screw extruder (TEX-44, manufactured by The Japan Steel Works, LTD.) under the following thermal decomposition conditions.

The thermally decomposed methyl methacrylate product containing methyl methacrylate was condensed using a heat exchanger to obtain a crude regenerated methyl methacrylate product.

Next, the crude regenerated methyl methacrylate product was purified using a rotary evaporator (R-300, manufactured by BUCHI) under the following purification conditions to obtain a regenerated methyl methacrylate product.

A content of regenerated methyl methacrylate contained in the obtained regenerated methyl methacrylate product was 97.3% by mass.

### (Thermal Decomposition Condition)

· Cylinder temperature: 450°C
· Rotation speed: 500 rpm
· Screw L/D: 60
· Supply amount of resin: 40 kg/hr

### (Purification Condition)

· Bath temperature: 50°C
· Circulating water temperature: 5°C
· Pressure: 90 mbar or more

### <Preparation Example 2>

### · Preparation of non-regenerated methyl methacrylate

Methacrylic acid and methanol were subjected to an ester reaction in a reactor to obtain methyl methacrylate.

Unreacted methanol was removed from the obtained methyl methacrylate product containing methyl methacrylate using an extraction column, and then purification was performed according to a conventional method using a plurality of distillation columns to obtain a non-regenerated methyl methacrylate product. A content of non-regenerated methyl methacrylate contained in the non-regenerated methyl methacrylate product was 99.99% by mass.

### <Example 1>

### · Preparation of Composition 1

5 parts by mass of the regenerated methyl methacrylate product obtained in Preparation Example 1 and 95 parts by mass of the non-regenerated methyl methacrylate product obtained in Preparation Example 2 were mixed to obtain Methyl methacrylate composition (hereinafter, simply referred to as "Composition") 1. Composition 1 obtained was a liquid form.

### · Preparation of Composition 1'

Composition 1 (99.83 parts by mass), sodium di-(2-ethylhexyl) sulfosuccinate (0.05 parts by mass) as a release agent, terpinolene (0.01 parts by mass) as a polymerization modifier, 2,2'-azobisisobutyronitrile (0.08 parts by mass) as a polymerization initiator, and 2-(2'-hydroxy-5'-methylphenyl)benzotriazole (0.01 parts by mass) as an ultraviolet absorber were mixed to obtain Composition 1' for forming a molded body (cast plate). Composition 1' obtained was a liquid form.

### · Preparation of Cast Plate 1

A vinyl chloride resin gasket having a thickness of 3.8 mm was sandwiched between two glass plates facing each other so as to prepare a cell defining a gap sealed by the vinyl chloride resin gasket and the two glass plates.

Composition 1' was injected into the gap inside the cell. The cell into which Composition 1' was injected was placed in an oven and subjected to heat treatment under heating conditions including the following Steps 1 to 7 in this order to polymerize Composition 1', thereby preparing cast plate 1, which is a 250 mm-square molded body having a thickness of 3 mm as a molded body of a methyl methacrylate-based polymer.

### (Heating Condition)

Step 1: Raise the temperature from room temperature to 68°C for 20 minutes.

Step 2: Hold the temperature at 68°C for 90 minutes.

Step 3: Lower the temperature from 68°C to 64°C for 20 minutes.

Step 4: Hold the temperature at 64°C for 90 minutes.

Step 5: Raise the temperature from 64°C to 123°C for 10 minutes.

Step 6: Hold the temperature at 123°C for 120 minutes.

Step 7: Lower the temperature from 123°C to room temperature for 90 minutes.

### · Measurement of 5% weight reduction temperature of cast plate 1

9.3 mg of a pulverized product obtained by pulverizing cast plate 1 so that a diameter or a length of each side was 0.5 mm or less was placed on an aluminum pan ("P/N SSC000E030 Open Sample Pan" manufactured by Hitachi High-Tech Science Corporation, diameter: 5 mm), and then a weight change was measured while the temperature was raised from 45°C to 520°C under the conditions of a nitrogen gas flow rate of 200 mL/min and a heating rate of 10°C/min using a thermogravimetric/differential thermal analyzer ("TG/DTA7200" manufactured by Hitachi High-Tech Science Corporation). As a result, the weight of the pulverized product decreased as the temperature increased. The weight of the pulverized product at the initial temperature of 45°C was taken as 100% by weight, and the temperature at which the weight of the pulverized product decreased to 95% by weight (5% weight reduction temperature) was determined. The results are shown in Table 1.

### · Measurement of glass transition temperature (Tmg) of cast plate 1

A glass transition temperature (°C) was measured in accordance with JIS-K7121 using, as a test piece, a pulverized product obtained by pulverizing cast plate 1 that is a molded body of a methyl methacrylate-based polymer so that a diameter or a length of each side was 0.5 mm or less.

Specifically, a differential scanning calorimeter ("DSC 7020", manufactured by Hitachi High-Tech Science Corporation) was used as a measurement apparatus for measuring the glass transition temperature, and a nitrogen gas flow rate was set to 50 mL/min. After the temperature was increased from room temperature (23°C) to 150°C at a rate of 20°C/min (primary temperature increase), the temperature was held at 150°C for 5 minutes, the test piece was completely melted, the temperature was decreased from 150°C to -35°C at a rate of 10°C/min, the temperature was held at -35°C for 1 minute, and the temperature was increased again to 210°C at a rate of 10°C/min (secondary temperature increase).

In the DSC curve obtained by the above temperature profile, an intersection (midpoint glass transition temperature) between a stepwise change partial curve at the time of the secondary temperature increase and a straight line equidistant from two base line extended lines in the vertical axis direction was defined as a glass transition temperature (°C). Three points per test piece were measured to calculate the average value, and the average value was defined as the glass transition temperature. The results are shown in Table 1.

### <Example 2>

### · Preparation of Composition 2

10 parts by mass of the regenerated methyl methacrylate product obtained in Preparation Example 1 and 90 parts by mass of the non-regenerated methyl methacrylate product obtained in Preparation Example 2 were mixed to obtain Composition 2. Composition 2 obtained was a liquid form.

Composition 2' was prepared in the same manner as that of Example 1 except that Composition 2 was used, and then Cast plate 2 was prepared, and a 5% weight reduction temperature and a glass transition temperature were measured. The results are shown in Table 1.

### <Example 3>

### · Preparation of Composition 3

30 parts by mass of the regenerated methyl methacrylate product obtained in Preparation Example 1 and 70 parts by mass of the non-regenerated methyl methacrylate product obtained in Preparation Example 2 were mixed to obtain Composition 3. Composition 3 obtained was a liquid form.

Composition 3' was prepared in the same manner as that of Example 1 except that Composition 3 was used, and then Cast plate 3 was prepared, and a 5% weight reduction temperature and a glass transition temperature were measured. The results are shown in Table 1.

### <Example 4>

### · Preparation of Composition 4

50 parts by mass of the regenerated methyl methacrylate product obtained in Preparation Example 1 and 50 parts by mass of the non-regenerated methyl methacrylate product obtained in Preparation Example 2 were mixed to obtain Composition 4. Composition 4 obtained was a liquid form.

Composition 4' was prepared in the same manner as that of Example 1 except that Composition 4 was used, and then Cast plate 4 was prepared, and a 5% weight reduction temperature and a glass transition temperature were measured. The results are shown in Table 1.

### <Example 5>

### · Preparation of Composition 5

80 parts by mass of the regenerated methyl methacrylate product obtained in Preparation Example 1 and 20 parts by mass of the non-regenerated methyl methacrylate product obtained in Preparation Example 2 were mixed to obtain Composition 5. Composition 5 obtained was a liquid form.

Composition 5' was prepared in the same manner as that of Example 1 except that Composition 5 was used, and then Cast plate 5 was prepared, and a 5% weight reduction temperature and a glass transition temperature were measured. The results are shown in Table 1.

### <Comparative Example 1>

### · Preparation of Composition C1

The liquid containing only of the regenerated methyl methacrylate product obtained in Preparation Example 1 was used as Composition C1.

Composition C1' was prepared in the same manner as that of Example 1 except that Composition C1 was used, and then Cast plate C1 was prepared, and a 5% weight reduction temperature and a glass transition temperature were measured. The results are shown in Table 1.

### <Reference Example 1>

### · Preparation of Composition R1

The liquid containing only the non-regenerated methyl methacrylate product obtained in Preparation Example 2 was used as Composition R1.

Composition R1' was prepared in the same manner as that of Example 1 except that Composition R1 was used, and then Cast plate R1 was prepared, and a 5% weight reduction temperature and a glass transition temperature were measured. The results are shown in Table 1.

### [Table 1]

**(Table 1)**

| | Regenerated methyl methacrylate product (parts by mass) | Non-regenerated methyl methacrylate product (parts by mass) | 5% weight reduction temperature (°C) | Glass transition temperature (°C) |
|---|---|---|---|---|
| Example 1 (Composition 1) | 5 | 95 | 292.2 | 114.7 |
| Example 2 (Composition 2) | 10 | 90 | 298.3 | 114.7 |
| Example 3 (Composition 3) | 30 | 70 | 310.9 | 113.9 |
| Example 4 (Composition 4) | 50 | 50 | 320.3 | 112.7 |
| Example 5 (Composition 5) | 80 | 20 | 325.8 | 111.7 |
| Comparative Example 1 (Composition C1) | 100 | 0 | 326.3 | 110.3 |
| Reference Example 1 (Composition R1) | 0 | 100 | 287.9 | 113.8 |

As is apparent from Table 1, in the cases of the molded bodies of the methyl methacrylate-based polymers according to Examples 1 to 5 satisfying the requirements of the present invention, excellent heat resistance was realized.

Here, a relationship between the proportion and the 5% weight reduction temperature or the glass transition temperature of regenerated methyl methacrylate will be described with reference to Figs. 1 and 2.

Fig. 1 is a graph showing the relationship between the proportion and the 5% weight reduction temperature of regenerated methyl methacrylate, and Fig. 2 is a graph showing the relationship between the proportion and the glass transition temperature of regenerated methyl methacrylate. The straight line (broken line) in each drawing is a straight line connecting values in a case where the proportion of the regenerated MMA is 0% (Reference Example 1) and in a case where the proportion of the regenerated MMA is 100% (Reference Example 2).

As illustrated in Fig. 1, the molded bodies of the methyl methacrylate-based polymers according to Examples 1 to 5 satisfying the requirements of the present invention showed an excellent 5% weight reduction temperature even when regenerated methyl methacrylate (regenerated methyl methacrylate product) was contained in the methyl methacrylate composition.

In addition, as illustrated in Fig. 2, in the cases of the molded bodies of the methyl methacrylate-based polymers according to Examples 1 to 5 satisfying the requirements of the present invention, an excellent glass transition temperature was realized.

As is apparent from Figs. 1 and 2, according to the molded bodies of the methyl methacrylate-based polymers of Examples 1 to 5 satisfying the requirements of the present invention, the 5% weight reduction temperature and the glass transition temperature were compatible with each other, and the balance between them was able to be improved.

Regarding the 5% weight reduction temperature, those skilled in the art would predict that the values in the case where the proportion of regenerated methyl methacrylate was 0% (Reference Example 1) and the case where the proportion of regenerated methyl methacrylate was 100% (Comparative Example 1) showed the behavior connected by the straight line (broken line in Fig. 1), the measured values of Examples 1 to 5 exceeded this prediction, and improvement of exceeding 10°C at the maximum was achieved. In addition, regarding the glass transition temperature, those skilled in the art would predict that the values in the case where the proportion of regenerated methyl methacrylate was 0% (Reference Example 1) and the case where the proportion of regenerated methyl methacrylate was 100% (Comparative Example 1) showed behavior connected by the straight line (broken line in Fig. 2) in consideration of the prediction values calculated from the FOX formula, and the measured values of Examples 1 to 5 were all considerably higher than this prediction. Specifically, the measured value exceeding the straight line tended to be obtained in the composition containing non-regenerated methyl methacrylate and regenerated methyl methacrylate except when the proportions of regenerated methyl methacrylate were 0% and 100%. This tendency was that a composition having a proportion of regenerated MMA of 5 to 80% had a measured value significantly exceeding the straight line.

### <Examples 6 and 7 and Comparative Example 2>

A regenerated methyl methacrylate product (regenerated MMA (A), content of regenerated methyl methacrylate: 96.7% by mass) was prepared under the same conditions as those of Preparation Example 1 except that the bath temperature and the pressure during purification were changed to 60°C and 170 mbar or more, respectively (Comparative Example 2). The regenerated MMA (A) and the non-regenerated MMA obtained in Preparation Example 2 were mixed at a mixing ratio shown in Table 2 to obtain each composition (liquid) (Examples 6 and 7). The 5% weight reduction temperature (hereinafter, referred to as T5) and the glass transition temperature (hereinafter, referred to as Tmg) of a molded body obtained by molding each composition by the method described above were measured under the conditions described above (Table 2).

### [Table 2]

**(Table 2)**

| | Mixing ratio | | T5 (°C) | Tmg (°C) |
|---|---|---|---|---|
| | Regenerated MMA (A) | Non-regenerated MMA | | |
| Example 6 | 5 | 95 | 296.5 | 115 |
| Example 7 | 80 | 20 | 323.9 | 108.2 |
| Comparative Example 2 | 100 | 0 | 323.8 | 106.2 |
| Reference Example 1 | 0 | 100 | 287.9 | 113.8 |

### <Examples 8 to 10 and Comparative Example 3>

A regenerated methyl methacrylate product (regenerated MMA (B): content of regenerated methyl methacrylate of 97.9% by mass) was prepared under the same conditions as those of Preparation Example 1 except that the polymethyl methacrylate resin as a raw material was changed to a PMMA waste material (illumination cover) (Comparative Example 3). The regenerated MMA (B) and the non-regenerated methyl methacrylate product (non-regenerated MMA) obtained in Preparation Example 2 were mixed at a mixing ratio shown in Table 3 to obtain each composition (liquid) (Examples 8 to 10). T5 and Tmg of a molded body obtained by molding each composition by the method described above were measured under the conditions described above (Table 3).

### [Table 3]

**(Table 3)**

| | Mixing ratio | | T5 (°C) | Tmg (°C) |
|---|---|---|---|---|
| | Regenerated MMA (B) | Non-regenerated MMA | | |
| Example 8 | 5 | 95 | 291.3 | 114.3 |
| Example 9 | 30 | 70 | 310.4 | 113.3 |
| Example 10 | 80 | 20 | 322.5 | 110.9 |
| Comparative Example 3 | 100 | 0 | 324.6 | 108.8 |
| Reference Example 1 | 0 | 100 | 287.9 | 113.8 |

### <Examples 11 and 12 and Comparative Example 4>

A regenerated methyl methacrylate product (regenerated MMA (C), content of regenerated methyl methacrylate: 97.8% by mass) was prepared under the same conditions as those of Preparation Example 1 except that the cylinder temperature during thermal decomposition was changed to 400°C (Comparative Example 4). The regenerated MMA (C) and the non-regenerated methyl methacrylate product (non-regenerated MMA) obtained in Preparation Example 2 were mixed at a mixing ratio shown in Table 4 to obtain each composition (liquid) (Examples 11 and 12). T5 and Tmg of a molded body obtained by molding each composition by the method described above were measured under the conditions described above (Table 4).

### [Table 4]

**(Table 4)**

| | Mixing ratio | | T5 (°C) | Tmg (°C) |
|---|---|---|---|---|
| | Regenerated MMA (C) | Non-regenerated MMA | | |
| Example 11 | 30 | 70 | 309.2 | 112.6 |
| Example 12 | 80 | 20 | 325.3 | 109.9 |
| Comparative Example 4 | 100 | 0 | 322.4 | 108.2 |
| Reference Example 1 | 0 | 100 | 287.9 | 113.8 |

### <Example 13 and Comparative Example 5>

A regenerated methyl methacrylate product (regenerated MMA (D), content of regenerated methyl methacrylate: 97.3% by mass) was prepared under the same conditions as those of Preparation Example 1 except that the cylinder temperature during thermal decomposition was changed to 480°C (Comparative Example 5). The regenerated MMA (D) and the non-regenerated methyl methacrylate product (non-regenerated MMA) obtained in Preparation Example 2 were mixed at a mixing ratio shown in Table 4 to obtain each composition (liquid) (Example 13). T5 and Tmg of a molded body obtained by molding each composition by the method described above were measured under the conditions described above (Table 5).

### [Table 5]

**(Table 5)**

| | Mixing ratio | | T5 (°C) | Tmg (°C) |
|---|---|---|---|---|
| | Regenerated MMA (D) | Non-regenerated MMA | | |
| Example 13 | 50 | 50 | 319.7 | 109.6 |
| Comparative Example 5 | 100 | 0 | 321.6 | 104.2 |
| Reference Example 1 | 0 | 100 | 287.9 | 113.8 |

As a result of plotting the results of T5 and Tg of each of the test examples in Figs. 3 and 4, T5 and Tg of the PMMA molded body obtained by mixing the regenerated MMA of each of Examples 1 to 5 of the present application **(•** in the drawings) and the PMMA molded body obtained by mixing the regenerated MMAs (A) to (D) (□, △, *, and - in the drawings, respectively) showed higher numerical values than the straight lines (broken lines) obtained by connecting T5 and Tmg of the molded body of Reference Example 1 (non-regenerated MMA 100%) and T5 of Comparative Example 2 and Tmg of Comparative Example 5 (maximum values of T5 and Tmg in Comparative Examples 2 to 5). In both Tmg and T5 (particularly, Tmg), the measured value exceeding the straight line tended to be obtained in the composition containing non-regenerated methyl methacrylate and regenerated methyl methacrylate except when the proportions of regenerated methyl methacrylate were 0% and 100%. This tendency was that a composition having a proportion of regenerated MMA of 5 to 80% had a measured value significantly exceeding the straight line.

### (Evaluation of Storage Stability of Composition)

Compositions 1 to 3 and R1 prepared in Examples 1 to 3, Comparative Example 1, and Reference Example 1 were subjected to a storage test in which the following Steps 1 to 7 were performed in this order. The storage test was carried out under accelerated conditions (80°C) in order to evaluate the stability after long-term storage.

Step 1: Into a lower part (container) of a pressure vessel ("TVS-N2 type" manufactured by Taiatsu Techno Corporation), 25 mL of the composition is injected.

Step 2: A packing is sandwiched between the upper part (upper lid) and the lower part of the pressure vessel, and the pressure vessel is sealed.

Step 3: Nitrogen is fed from the tip (piping) of the upper part of the pressure vessel, and sealed in a state where the internal pressure is 0.2 MPa, and it is confirmed whether the internal pressure does not change for 1 minute.

Step 4: A closing plug is attached to the tip of the upper part of the pressure vessel.

Step 5: The pressure vessel is placed in an oil bath set at 80°C.

Step 6: The pressure vessel is stored in the oil bath for 24 hours.

Step 7: After 24 hours have elapsed, the pressure vessel is removed from the oil bath.

The composition (4 mL) after the storage test was placed in a quartz glass cell (T-1, manufactured by DAICO MFG CO., Ltd.) having a size of 10 mm in length × 10 mm in width × 45 mm in height. Using the composition placed in the quartz glass cell, a yellow index (YI value) at an optical path length of 10 mm was determined according to the method described in JIS K7373 (Table 6). The measurement was performed using a spectrophotometer ("Hitachi Spectrophotometer U-4000" manufactured by Hitachi High-Tech Fielding Corporation) under the following conditions. As the YI value is smaller, it can be determined that the yellow discoloration can be suppressed even after storage.

### (YI Measurement Condition)

Light source: C light source
Field of view: 2 degrees Calculation Formula: YI = (1.2769 × X - 1.0592 × Z)/Y × 100

### [Table 6]

**(Table 6)**

| | | Reference Example 1 (Composition R1) | Example 1 (Composition 1) | Example 2 (Composition 2) | Example 3 (Composition 3) | Comparative Example 1 (Composition C1) |
|---|---|---|---|---|---|---|
| Heating condition | - | 80°C 25 hr | 80°C 26 hr | 80°C 27 hr | 80°C 25 hr | 80°C 25 hr |
| Non-regenerated MMA | % | 100 | 70 | 50 | 30 | 0 |
| Regenerated MMA | % | 0 | 30 | 50 | 70 | 100 |
| X | | 97.6437 | 97.9527 | 97.5874 | 97.813 | 96.9996 |
| Y | | 99.6099 | 99.835 | 99.4988 | 99.7417 | 98.9066 |
| Z | | 117.552 | 118.02 | 117.725 | 118.005 | 116.597 |
| Yellow index YI | | 0.2 | 0.1 | -0.1 | -0.1 | 0.4 |

As compared with Compositions R1 and C1 of Reference Example 1 and Comparative Example 1, Compositions 1 to 3 of Examples 1 to 3 had a low YI value after the storage test, indicating that the yellow change after storage was suppressed (Table 6).

## Claims

1. A methyl methacrylate composition comprising
regenerated methyl methacrylate and non-regenerated methyl methacrylate,
wherein a content of the regenerated methyl methacrylate is 0.5 to 99.5% by mass, and a content of the non-regenerated methyl methacrylate is 0.5 to 99.5% by mass.

2. The methyl methacrylate composition according to claim 1, wherein the regenerated methyl methacrylate is regenerated methyl methacrylate derived from chemical recycling.

3. The methyl methacrylate composition according to claim 1 or 2, wherein the content of the regenerated methyl methacrylate is 5 to 40% by mass, and the content of the non-regenerated methyl methacrylate is 60 to 95% by mass.

4. The methyl methacrylate composition according to claim 1 or 2, further comprising an alkyl acrylate.

5. A method of producing a methyl methacrylate-based polymer, the method comprising polymerizing the methyl methacrylate composition according to claim 1 or 2.

6. A methyl methacrylate-based polymer obtained by polymerizing the methyl methacrylate composition according to claim 1 or 2.

7. A methyl methacrylate-based polymer comprising a structural unit derived from regenerated methyl methacrylate and a structural unit derived from non-regenerated methyl methacrylate, wherein a content of the structural unit derived from regenerated methyl methacrylate is 0.5 to 99.5% by mass, and a content of the structural unit derived from non-regenerated methyl methacrylate is 0.5 to 99.5% by mass.
